# EUROPEAN PATENT SPECIFICATION

(11) **EP 1 465 682 B1**
(45) Date of publication and mention of the grant of the patent: **29.04.2009**
(21) Application number: 02803959.2
(22) Date of filing: 05.09.2002
(51) Int. Cl.: A61L 29/18, A61L 31/18

(54) **MEDICAL DEVICES WITH MAGNETIC RESONANCE VISIBILITY ENHANCING MATERIAL**
MEDIZINISCHE ARTIKEL MIT ZUSÄTZEN ZUR ERHÖHUNG DER NMR-SICHTBARKEIT
DISPOSITFS MEDICAUX DOTES D'UNE MATIERE AMELIORANT LA VISIBILITE DE RESONANCE MAGNETIQUE

(30) Priority: 27.11.2001 US 995528
(43) Date of publication of application: 13.10.2004
(73) Proprietor: Boston Scientific Limited, St. Michael, Barbados, West Indies (BB)
(72) Inventor: ZHONG, Sheng-Ping, Shrewsbury, MA 01545 (US); SAHATJIAN, Ronald, A., Lexington, MA 02420-2121 (US)
(74) Representative: Peterreins, Frank
(86) International application number: PCT/US2002/028202
(87) International publication number: WO 2003/045462

(56) References cited:
- EP-A- 0 701 836
- EP-A- 0 775 500
- EP-A- 1 206 945
- WO-A-01/81460
- WO-A-02/22186
- WO-A-94/08629
- WO-A-94/23782
- WO-A-99/60920
- US-A- 4 989 608
- JIANG X ET AL: "NOVEL MAGNETIC RESONANCE SIGNAL ENHANCING COATING MATERIAL" ADVANCED MATERIALS, VCH VERLAGSGESELLSCHAFT, WEINHEIM, DE, vol. 13, no. 7, 4 April 2001 (2001-04-04), pages 490-493, XP001044106 ISSN: 0935-9648

## Description

### BACKGROUND OF THE INVENTION

The present invention relates generally to intralumenal devices for use in magnetic resonance imaging. More particularly, the present invention relates to intralumenal devices that incorporate a magnetic resonance visibility enhancing material, the devices being adapted for use in magnetic resonance imaging.

Magnetic resonance imaging (MRI) is a non-invasive medical procedure that utilizes magnets and radio waves to produce a picture of the inside of a body. An MRI scanner is capable of producing pictures of the inside of a body without exposing the body to ionizing radiation (X-rays). In addition, MRI scans can see through bone and provide detailed pictures of soft body tissues.

A typical MRI scanner includes a magnet that is utilized to create a strong homogeneous magnetic field. A patient is placed into or proximate the magnet. The strong magnetic field causes atoms within the patient's body to align. A radio wave is directed at the patient's body, triggering atoms within the patient's body cavity tissues to emit radio waves of their own. These return radio waves create signals (resonance signals) that are detected by the scanner at numerous angles around the patient's body. The signals are sent to a computer that processes the information and compiles an image or images. Typically, although not necessarily, the images are in the form of 2-dimensional "slice" images.

Some MRI applications utilize a contrast medium, also known as a contrast agent. Typically, a contrast medium contains paramagnetic material and is injected into the bloodstream of a patient. The contrast medium alters the inherent response to magnetic fields of atoms contained within proximately located blood and body tissues. In this way, contrast mediums may enable blood flow to be tracked and/or a greater sensitivity for MRI detection and characterization of different body tissues.

Gadolinium, a periodic table element, is an example of a material that has been utilized within the context of contrast mediums. Gadolinium has eight unpaired electrons in its outer shell, which causes it to be paramagnetic in nature. Gadolinium, when bound to a chelator retains paramagnetic properties and is relatively safe for exposure to the body.

In some MRI applications, a gadolinium-based contrast medium is introduced into a body through intravenous injection. When injected in the bloodstream of a patient, the gadolinium alters the inherent response to magnetic fields of atoms contained within proximately located blood and body tissues. In particular, the gadolinium shortens the relaxation time of atoms contained in the blood and tissue that are in regions proximate to the gadolinium molecules. During the MRI process, this shortening of relaxation time caused by the gadolinium-based contrast medium translates into images that are highlighted or brightened in the areas of atoms demonstrating the shortened relaxation.

Within some MRI applications, catheters and other intralumenal devices may be inserted into a body during the MRI process. An ability to locate, trace and position such devices in their intralumenal environments is desirable. A material similar to a contrast medium (i.e., a paramagnetic material) may be directly disposed on at least a portion of an intralumenal device to enhance MRI visibility. Under the typical environmental conditions associated with the intralumenal manipulation of a medical device, exposure of the intralumenal device to stationary body tissue and fluid is limited. As a result, interaction between fluid/tissue and the material disposed on the intralumenal device is also limited.

The present invention addresses at least one of these and other problems and offers advantages over the prior art.

WO 94/23782 discloses a catheter including paramagnetic ionic particles combined with a polymeric material. The combined particles and polymeric material may then be formed into a tube by conventional techniques, such as extrusion.

WO 01/81460 relates to an hydrophilic biomaterial incorporating paramagnetic particles which could be used as a coating, inter alia, of a catheter.

US4989608 relates to a catheter wherein a suitable ferromagnetic material is incorporated into the plastic by mixing the ferromagnetic particles into the plastic as it is being extruded.

EP 0775500 relates to a catheter wherein sections of paramagnetic material are alternated to sections of basic material. The body sections can for instance be determined during the extrusion of the basic body by means of the controlled supply of plastic material comprising para and/or ferromagnetic material, alternated with plastic material free of those materials.

EP 0701836 relates to a catheter comprising a tube-like body extruded from a plastic material comprising a filler of paramagnetic material. As a result the position of the catheter inside the body of a patient can be made visible on an NMR screen.

Reference "NOVEL MAGNETIC RESONANCE SIGNAL ENHANCING COATING MATERIAL", ADVANCED MATERIALS, VCH VERLAGSGESELLSCHAFT, WEINHEIM, DE, Vol. 13, no. 7, 4 April 2001, pages 490-493 discloses catheters having a surface modified polymer by covalent linking chelator molecules for a magnetic cation.

WO 02/22186 relates to medical devices, including catheters, where the medical device can have a surface coating including a lubricious polymer. In one embodiment a polymeric substrate and the magnetic susceptible agent are co-extruded to form the medical device. After extrusion, the medical device can be coated with lubricious polymers.

EP 1206945 discloses a catheter comprising a polymer and a paramagnetic substance.

### SUMMARY OF THE INVENTION

The present invention generally pertains to intralumenal devices adapted to be advanced through a patient during a magnetic resonance imaging procedure. In particular, the present invention provides a method for constructing a medical device and a medical device according to the claims. These and various other features, as well as advantages that characterize the present invention, will be apparent upon a reading of the following detailed description and review of the associated drawings.

### BRIEF DESCRIPTION OF THE DRAWINGS

FIG. 1 is a partial block diagram of an illustrative magnetic resonance imaging system in which illustrative embodiments of the present invention can be employed.
FIG. 2 is a side view of a magnetic resonance catheter in accordance with an illustrative embodiment of the present invention.
FIG. 3 PRIOR ART is an enlarged cross-sectional view of a catheter.
FIG. 4 is an enlarged cross-sectional view of the catheter shown in FIG. 2, in accordance with an illustrative embodiment of the present invention.

### DETAILED DESCRIPTION OF ILLUSTRATIVE EMBODIMENTS

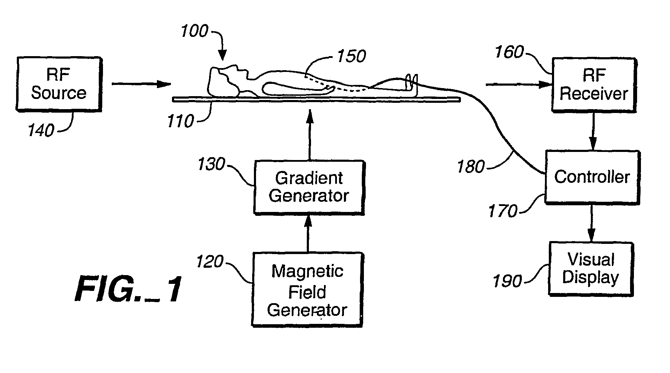

FIG. 1 is a partial block diagram of an illustrative magnetic resonance imaging system in which embodiments of the present invention could be employed. In FIG. 1, subject 100 on support table 110 is placed in a homogeneous magnetic field generated by magnetic field generator 120. Magnetic field generator 120 typically comprises a cylindrical magnet adapted to receive subject 100. Magnetic field gradient generator 130 creates magnetic field gradients of predetermined strength in three mutually orthogonal directions at predetermined times. Magnetic field gradient generator 130 is illustratively comprised of a set of cylindrical coils concentrically positioned within magnetic field generator 120. A region of subject 100 into which a device 150, shown as a catheter, is inserted, is located in the approximate center of the bore of magnetic 120. Illustratively, device 150 could be a guidewire or some other intralumenal device.

RF source 140 radiates pulsed radio frequency energy into subject 100 and device 150 at predetermined times and with sufficient power at a predetermined frequency to influence nuclear magnetic spins in a fashion well known to those skilled in the art. The influence on the spins causes them to resonate at the Larmor frequency. The Larmor frequency for each spin is directly proportional to the strength of the magnetic field experienced by the spin. This field strength is the sum of the static magnetic field generated by magnetic field generator 120 and the local field generated by magnetic field gradient generator 130. In an illustrative embodiment, RF source 140 is a cylindrical external coil that surrounds the region of interest of subject 100. Such an external coil can have a diameter sufficient to encompass the entire subject 100. Other geometries, such as smaller cylinders specifically designed for imaging the head or an extremity can be used instead. Non-cylindrical external coils such as surface coils may alternatively be used.

Device 150 is inserted into subject 100 by an operator. Illustratively, device 150 may alternatively be a guidewire, a catheter, an abation device or a similar recanalization device, or some other intralumenal device.

In accordance with one embodiment, but not by limitation, device 150 illustratively includes an RF antenna that detects magnetic resonance (MR) signals generated in both the subject and the device 150 itself in response to the radio frequency field created by RF source 140. Since the internal device antenna is small, the region of sensitivity is also small. Consequently, the detected signals have Larmor frequencies, which arise only from the strength of the magnetic field in the proximate vicinity of the antenna. The signals detected by the device antenna are sent to imaging and tracking controller unit 170 via conductor 180. It should be emphasized that device 150 need not incorporate a device antenna to be within the scope of the present invention.

In accordance with one embodiment, medical devices (such as but not limited to catheters) with the below-described embodiments of integrated magnetic resonance visibility enhancing material can be utilized in combination with a device antenna to assist in tracking and locating the device antenna. This combination of features illustratively provides both passive and active image enhancement.

External RF receiver 160 illustratively detects RF signals emitted by the subject in response to the radio frequency field created by RF source 140. In an illustrative embodiment, external RF receiver 160 is a cylindrical external coil that surrounds the region of interest of subject 100. Such an external coil can have a diameter sufficient to have a compass the entire subject 100. Other geometries, such as smaller cylinders specifically designed for imaging the head or an extremity can be used instead. Non-cylindrical external coils, such as surface coils, may alternatively be used. External RF receiver 160 can share some or all of its structure with RF source 140 or can have a structure entirely independent of RF source 140. The region of sensitivity of RF receiver 160 is larger than that of the device antenna and can encompass the entire subject 100 or a specific region of subject 100. However, the resolution which can be obtained from external RF receiver 160 is less than that which can be achieved with the device antenna. The RF signals detected by external RF receiver 160 are sent to imaging and tracking controller unit 170 where they are analyzed together with any RF signals detected by the device antenna.

In accordance with an embodiment of the present invention, external RF receiver 160 detects RF signals emitted by device 150 in response to the radio frequency field created by RF source 140. Illustratively, these signals are sent to imaging and tracking controller unit 170 where they are translated into images of device 150. In accordance with one embodiment, the position of device 150 is determined in imaging and tracking controller unit 170 and is displayed on display means 190. In one illustrative embodiment, the position of device 150 is displayed on display means 190 by superposition of a graphic symbol on a conventional MR image obtained by external RF receiver 160. Alternatively, images may be acquired by external RF receiver 160 prior to initiating tracking and a symbol representing the location of the tracked device be superimposed on the previously acquired image. Alternative embodiments of the invention display the position of the device numerically or as a graphic symbol without reference to a diagnostic image.

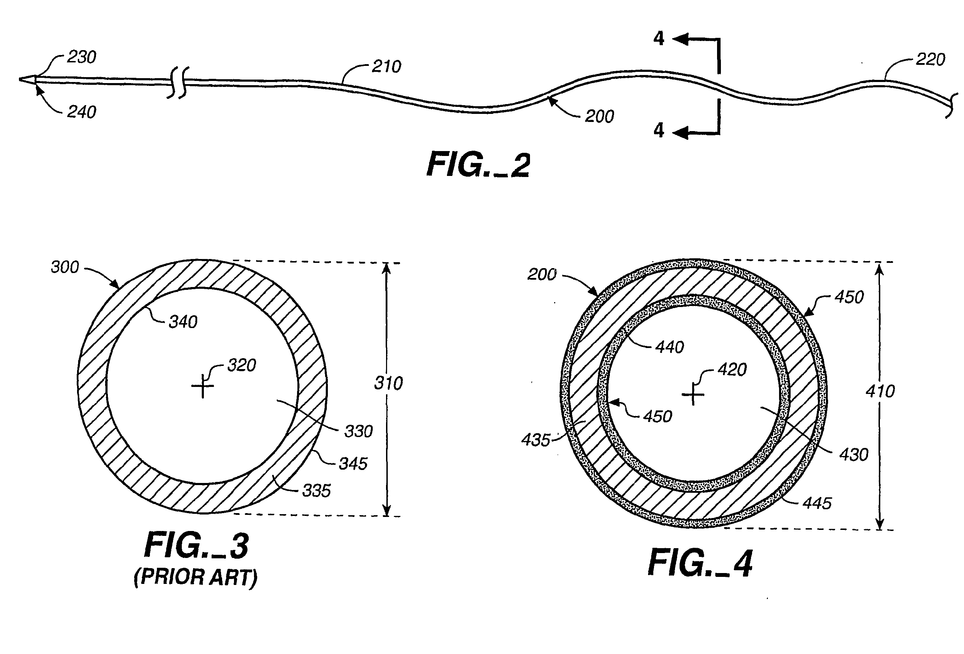

FIG. 2 is side view of one illustrative embodiment of a device that could be utilized similar to device 150 described above in relation to FIG. 1. More particularly, FIG. 2 is a side view of a magnetic resonance catheter 200 (MR catheter 200), in accordance with an illustrative embodiment of the present invention. MR catheter 200 includes an elongated body 210 having a proximal end 220 and a distal end 230. Illustratively, an antenna 240 is optionally disposed proximate distal end 230 and operates as described above in relation to FIG. 1.

FIG. 3 PRIOR ART is an enlarged cross-sectional view of a typical catheter identified as catheter 300. Catheter 300 includes a circumference 310 and an axis 320. Catheter 300 also includes a lumen 330. Lumen 330 is illustratively formed and defined by a coaxial, tubular catheter body 335 (body 335). Body 335 is typically constructed of a flexible polymeric material or some other flexible material. Body 335 includes an optional coaxial layer 340 of undercoat material. Optional layer 340 is typically constructed of a layer of material such as urethane, PVC, polyamide, silicon, PTFE, polyurethane or some other similar material. Body 335 includes an optional coaxial outer protective layer 345. Any of the body 335, optional layer 340 and optional layer 345 may be formed with additional layers. For example, a reinforcement layer may be included to improve certain mechanical characteristics. FIG. 3 PRIOR ART is provided for comparative purposes to better illustrate illustrative embodiments of the present invention.

FIG. 4, in accordance with an embodiment of the present invention, is an enlarged cross-sectional view of MR catheter 200 taken along line 4--4 in FIG. 2. As is illustrated in FIG. 4, MR catheter 200 includes a circumference 410 and an axis 420, which each illustratively extend at least from proximal end 220 to distal end 230 (FIG. 2). The MR catheter 200 also includes a lumen 430 that also illustratively extends between ends 220 and 230. It should be noted that catheters having additional lumens (multi-lumen catheter) should be considered within the scope of the present invention.

With further reference to FIG. 4, lumen 430 is illustratively formed and defined by a coaxially formed tubular catheter body 435 (body 435). In accordance with one embodiment, body 435 is constructed of a flexible polymeric material. Body 435, however, may be constructed of other materials without departing from the scope of the present invention.

Body 435 includes an optional coaxial layer 440 of undercoat material. Illustratively, optional layer 440 could be constructed of a layer of material such as urethane, PVC, polyamide, silicon, PTFE, polyurethane or some other material. Body 435 also includes an optional coaxial outer protective layer 445. Optional layer 445 could illustratively be some form of a lubricious coating. It should be noted that, without departing from the scope of the present invention, any of the body 435, optional layer 440 and optional layer 445 could illustratively be formed with additional layers. For example, a reinforcement layer may be included to improve certain mechanical characteristics. In accordance with one embodiment, a reinforcement layer is included and is configured to operate as an internal RF antenna or a device antenna and provides active MRI image enhancement.

Still referring to FIG. 4, the MR catheter 200, in accordance with an embodiment of the present invention, further includes magnetic resonance visibility enhancing material 450 (MR material 450) disposed on the inside of body 435 (proximate lumen 430) and on the outside of body 435. It should be noted that, in accordance with embodiments of the present invention, magnetic resonance material 450 could be disposed either on the inside of body 435 or on the outside of body 435. In addition, MR material 450 need not necessarily be coaxially continuous as illustrated. Also, MR material 450 could illustratively be in a general layer that is thinner or thicker than illustrated without departing from the scope of the present invention. The precise configuration details of material 450 are application dependent and will vary depending on a particular desired functional outcome.

The MR material 450 is illustratively disposed on a surface or surfaces of catheter 200. MR material 450 comprises a hydrophilic polymer. MR material 450 comprises a hydrophilic polymer having a magnetic resonance material incorporated therein. The magnetic resonance material may illustratively be incorporated into the hydrophilic polymer by traditional means, such as compounding or blending. The incorporated magnetic resonance materials may be or include paramagnetic metal salt, paramagnetic particles (i.e., super-magnetic iron oxide, dysprosium, etc.), paramagnetic metal chelate, material, gadolinium, Gd-DTPA (Gadolinium diethylenetriaminepentaacetic acid), or some other paramagnetic material. In accordance with another embodiment, a soluble gadolinium salt is incorporated or cross-linked into the hydrophilic polymer matrix. Illustratively, the soluble gadolinium salt becomes part of the hydrophilic polymer.

In accordance with an embodiment of the present invention, upon contact with body fluid when catheter 200 is in use, the hydrophilic material in MR material 450 gets hydrated in a controlled fashion. In accordance with one embodiment, MR material 450 is pre-soaked or pre-hydrated (illustratively but not necessarily with water or saline and illustratively but not necessarily for five minutes) before catheter 200 is inserted into the patient. The hydrophilic polymer in material 450 influences the relaxation time of the atoms captured within the hydrophilic polymer (i.e., the relaxation time is shortened) and thereby enhances the MRI visibility of catheter 200. Illustratively, the hydrophilic polymer modulates the relaxation time of the captured atoms (i.e., shortens t1 and/or t2, which are relaxation factors known in the art) to enable creation of an MR image of the catheter. In accordance with one embodiment, as the result of the described influenced relaxation time, catheter 200 will essentially "light up" under MRI.

Paramagnetic material is incorporated into the hydrophilic polymer to enhance MRI visibility. Illustratively, the paramagnetic material in material 450 influences the relaxation time of the hydrated polymer (i.e., the relaxation time is shortened) and thereby enhances the MRI visibility of catheter 200. In accordance with one embodiment, as the result of a shortened relaxation time, catheter 200 will essentially "light up" under MRI. The paramagnetic material illustratively might be, but is not limited to, paramagnetic ionic material.

The MR material 450 can illustratively be applied to a surface of catheter 200 (or some other medical device) in a variety of ways. A variety of hydrophilic polymers having a variety of different attributes and physical characteristics could be utilized in the context of the present invention.

Hydrophilic polymers may demonstrate different physical characteristics that enable different modes of integration or attachment with a medical device. For example, some hydrophilic polymers could illustratively be integrated or attached to catheter 200 utilizing an extrusion process. Some extrudable hydrophilic polymers may inherently demonstrate particularly desirable mechanical characteristics (desirable tensile strength, durability, etc.) following an application to catheter 200 utilizing an extrusion process. Other hydrophilic polymers may be less desirable in terms of inherent mechanical characteristics.

A hydrophilic polymer is applied to catheter 200 through co-extrusion with a structural polymer. The structural polymer provides desirable mechanical properties while the hydrophilic polymer provides magnetic resonance visibility. In accordance with one embodiment, this co-extruded hydrophilic material can be cross-linked to enhance its durability. Radiation, or other chemical means can illustratively be utilized to achieve the cross-linking. In accordance with another embodiment, a hydrophilic polymer is compounded or blended with a structural polymer. The compounded or blended polymers are applied to catheter 200 and provide a material having structurally beneficial properties.

A hydrophilic polymer, along with incorporated paramagnetic components (i.e., paramagnetic metal salt, paramagnetic metal chelate, paramagnetic metal complex, other paramagnetic ionic material, paramagnetic particles, etc), is applied to catheter 200 through co-extrusion with a structural polymer. The structural polymer provides desirable mechanical properties while the hydrophilic polymer, and its incorporated components, provide magnetic resonance visibility. In accordance with one embodiment, this co-extruded hydrophilic material can be cross-linked to enhance its durability. Radiation, or other chemical means can illustratively be utilized to achieve the cross-linking. In accordance with another embodiment, a hydrophilic polymer, along with incorporated paramagnetic components, is compounded or blended with a structural polymer. The compounded or blended polymers are applied to catheter 200 and provide a material having structurally beneficial properties.

Catheter 200 is generally manufactured or constructed utilizing a structural polymer having a hydrophilic polymer compounded therein. In other words, the structural polymer is what generally gives shape to catheter 200, and it has a hydrophilic polymer compounded therein. In essence, catheter 200 is manufactured or constructed to inherently include material 450. This method of integration/attachment stands in contrast to the incorporation of a hydrophilic polymer with a structural polymer that is itself attached or integrated with catheter 200.

Catheter 200 is generally manufactured or constructed utilizing a structural polymer having a hydrophilic polymer, along with incorporated paramagnetic components (i.e., paramagnetic metal salt, paramagnetic metal chelate, paramagnetic metal complex, other paramagnetic ionic material, paramagnetic particles, etc), compounded therein. In other words, the structural polymer is what generally gives shape to catheter 200, and it has a hydrophilic polymer and associated paramagnetic polymers compounded therein. In essence, catheter 200 is manufactured or constructed to inherently include material 450. This method of integration/attachment stands in contrast to an incorporation of components with a structural polymer that is itself attached or integrated with catheter 200.

The above-described extrusion, co-extrusion and general compounding applications of material 450 are alternatives beyond coating to provide device 200 with the described magnetic resonance characteristics. In many instances, compared to coating, extrusion, co-extrusion or general compounding can be quicker and cheaper than coating or dip coating.

Referring to FIG. 4, as was previously mentioned, an MR material 450 may be disposed on the inside of body 435 (proximate lumen 430) and/or on the outside of body 435. Illustratively, extrusion or co-extrusion provides a relatively simple application means for attaching an MR material 450 to the inside of body 435 (the tubular inside of catheter 200). Placement of MR material 450 within or on the inside of body 435 has certain illustrative advantages. For example, during use of device 200, there generally may be less fluid exchange in the inner lumen of body 435 than on the external or outside surface of body 435. In the context of embodiments wherein paramagnetic ions are incorporated with a hydrophilic polymer, losses of paramagnetic material from the hydrophilic polymer could be decreased in the case of placement of MR material within or on the inside of body 435. Such placement might enable a better longevity of the magnetic resonance visibility effects.

Examples of hydrophilic polymers suitable for extrusion or co-extrusion are: polyethylene oxide (PEO), polypropylene oxide (PPO), polyvinylpyrrolidone (PVP), hydrophilic polyurethanes, polypropylene, starches, polycarboxylic acids, cellulosic polymers, gelatin, maleic anhydride polymers, polyamides, polyvinyl alcohols, polyacrylic acid, and polyethylene oxides. Other hydrophilic polymers, however, should be considered within the scope of the present invention. Examples of structural polymers suitable for co-extrusion are: Nylon, PEBAX, polyurethane, polyethylene, PEEK, polyimide, polyester-amide copolymer and polyether-amide copolymer. Other structural polymers, however, should be considered within the scope of the present invention.

Although the present description has been described in the context of catheter 200, the present invention could just as easily be applied in the context of other medical devices, and in particular, in the context of other intralumenal medical devices. For example, the above-described material configurations and attachment/integration methods could just as easily be applied to produce implant devices, guide wires, catheters of many types (including vascular and non-vascular and esophogeal catheters), ablation devices or any other medical device having an enhanced MRI visibility. In accordance with one embodiment, the above-described material configurations and attachment/integration methods are applied to produce balloons (i.e., angioplasty balloons) having an enhanced MRI visibility. In the context of tubular devices, the above-described MR visibility enhancement material could illustratively be extruded or co-extruded on an outer surface, inner surface or both surfaces. Similarly, for non-tubular devices, the material could be extruded or co-extruded on one or both sides of a surface.

In conclusion, the present invention relates to a method of creating and applying a magnetic resonance visibility enhancing material to a medical device through, for example, compounding (i.e., compounding elements into structural polymer that forms a given medical device), extrusion or co-extrusion process. The material enables the device to be visible under MRI. The material includes a hydrophilic polymer and paramagnetic material. The devices may be catheters, such as neuro-interventional micro-catheters, or any other appropriate MRI medical device. The devices may illustratively enable physicians to perform procedures under an open MRI system, instead of under X-ray. The devices illustratively reduce radiation exposure to both physicians and patients. The described MRI materials illustratively help the tracking and positioning of devices. The devices may illustratively be implant devices, so physicians can check/track the implants under MRI with 3D images.

## Claims

1. A method of constructing a medical device, comprising:
providing a medical device (200); and
integrating a hydrophilic polymer that incorporates a substance having a plurality of paramagnetic ions with the medical device (200), **characterized in that** the integrating of the hydrophilic polymer comprises:
co-extruding onto a surface of the medical device (200) a structural polymer in combination with a hydrophilic polymer that incorporates a substance having a plurality of paramagnetic ions.

2. The method of claim 1, wherein the integrating of the hydrophilic polymer with the medical device (200) comprises:
integrating the hydrophilic polymer with a balloon device.

3. The method of claim 1, further comprising:
applying at least one final coating to the medical device (200) so as to leave exposed at least one portion of the hydrophilic polymer and the plurality of paramagnetic ions incorporated therein.

4. The method of claim 3, wherein the applying the at least one final coating to the medical device (200) comprises:
applying a lubricious coating to the medical device (200).

5. The method of claim 3, wherein the applying the at least on final coating to the medical device (200) comprises:
applying a coating that contains a therapeutic agent to the medical device (200).

6. An elongated medical device for intralumenal manipulation during a process of magnetic resonance imaging, comprising:
an elongated body (435); and
an extrusion material that includes a hydrophilic polymer that incorporates a substance having a plurality of paramagnetic particles, the extrusion material being integrated with the elongated body and configured to enhance magnetic resonance visibility during said process of magnetic resonance imaging;
wherein the extrusion material is a co-extrusion material that comprises:
a first co-extrusion component comprising a hydrophilic polymer that incorporates a substance having a plurality of paramagnetic particles, the first co-extrusion component being configured to enhance magnetic resonance visibility; and
a second co-extrusion component comprising a structural polymer, the second co-extrusion component being configured to provide structural support.

7. The elongated medical device of claim 6, wherein the plurality of paramagnetic particles comprises super-magnetic iron oxide.

8. The elongated medical device of claim 6, wherein the plurality of paramagnetic particles comprises dysprosium oxide.

## Patentansprüche

1. Verfahren zur Konstruktion einer medizinischen Vorrichtung, umfassend:
Bereitstellen einer medizinischen Vorrichtung (200); und
Integrieren eines hydrophilen Polymers, das eine Substanz mit einer Mehrzahl paramagnetischer Ionen in die medizinische Vorrichtung (200) einbringt,
**dadurch gekennzeichnet, dass** das Integrieren des hydrophilen Polymers umfasst:
Co-Extrudieren eines strukturellen Polymers in Kombination mit einem hydrophilen Polymer, das eine Substanz mit einer Mehrzahl von paramagnetischen Ionen beinhaltet, auf eine Oberfläche der medizinischen Vorrichtung (200).

2. Verfahren nach Anspruch 1, wobei das Integrieren des hydrophilen Polymers in die medizinische Vorrichtung (200) umfasst:
Integrieren des hydrophilen Polymers in eine Ballon-Vorrichtung.

3. Verfahren nach Anspruch 1, weiter umfassend:
Aufbringen mindestens einer letzten Beschichtung auf die medizinische Vorrichtung (200), um mindestens einen Abschnitt des hydrophilen Polymers und der Mehrzahl paramagnetischer Ionen, die darin enthalten sind, exponiert zu lassen.

4. Verfahren nach Anspruch 3, wobei das Aufbringen der mindestens einen letzten Beschichtung auf die medizinische Vorrichtung (200) umfasst:
Aufbringen einer schlüpfrigen Beschichtung auf die medizinische Vorrichtung (200).

5. Verfahren nach Anspruch 3, wobei das Aufbringen der mindestens einen letzten Beschichtung auf die medizinische Vorrichtung (200) umfasst:
Aufbringen einer Beschichtung, die einen therapeutischen Wirkstoff beinhaltet, auf die medizinische Vorrichtung (200).

6. Eine längliche medizinische Vorrichtung für intraluminale Manipulation während eines Prozesses der Magnetresonanztomografie, umfassend:
einen länglichen Körper (435); und
ein Extrusionsmaterial, das ein hydrophiles Polymer enthält, das eine Substanz mit einer Mehrzahl paramagnetischer Partikel beinhaltet, wobei das Extrusionsmaterial mit dem länglichen Körper integriert ist und konfiguriert, um magnetische Resonanzsichtbarkeit während des Prozesses der Magnetresonanztomografie zu verbessern;
wobei das Extrusionsmaterial ein Co-Extrusionsmaterial ist, welches umfasst:
eine erste Co-Extrusionskomponente, umfassend ein hydrophiles Polymer, das eine Substanz mit einer Mehrzahl paramagnetischer Partikel beinhaltet, wobei die erste Co-Extrusionskomponente konfiguriert ist, um magnetische Resonanzsichtbarkeit zu verbessern; und
eine zweite Co-Extrusionskomponente, umfassend ein strukturelles Polymer, wobei die zweite Co-Extrusionskomponente konfiguriert ist, um strukturelle Unterstützung bereitzustellen.

7. Längliche medizinische Vorrichtung nach Anspruch 6, wobei die Mehrzahl paramagnetischer Partikel super-magnetisches Eisenoxid umfasst.

8. Längliche medizinische Vorrichtung nach Anspruch 6, wobei die Mehrzahl paramagnetischer Partikel Disprosiumoxid umfasst.

## Revendications

1. Procédé de construction d'un dispositif médical comprenant :
la mise à disposition d'un dispositif médical (200) ; et
l'intégration d'un polymère hydrophile qui comprend une substance ayant une pluralité d'ions paramagnétiques au dispositif médical (200),
**caractérisé en ce que** l'intégration du polymère hydrophile comprend :
la co-extrusion sur une surface du dispositif médical (200) d'un polymère structural en combinaison avec un polymère hydrophile qui comprend une substance ayant une pluralité d'ions paramagnétiques.

2. Procédé selon la revendication 1, dans lequel l'intégration du polymère hydrophile avec le dispositif médical (200) comprend :
l'intégration du polymère hydrophile à un dispositif à ballon.

3. Procédé selon la revendication 1, comprenant en outre :
l'application d'au moins un revêtement final sur le dispositif médical (200) de façon à laisser exposée au moins une partie du polymère hydrophile et la pluralité d'ions paramagnétiques incorporés dans celui-ci.

4. Procédé selon la revendication 3, dans lequel l'application d'au moins un revêtement final sur le dispositif médical (200) comprend :
l'application d'un revêtement de lubrification sur le dispositif médical (200).

5. Procédé selon la revendication 3, dans lequel l'application d'au moins un revêtement final sur le dispositif médical (200) comprend :
l'application d'un revêtement qui contient un agent thérapeutique sur le dispositif médical (200).

6. Dispositif médical allongé pour une manipulation intraluminale pendant un processus d'imagerie par résonance magnétique, comprenant :
un corps allongé (435) ; et
un matériau d'extrusion qui comprend un polymère hydrophile qui comprend une substance ayant une pluralité de particules paramagnétiques, le matériau d'extrusion étant intégré avec le corps allongé et configuré de façon à renforcer la visibilité par résonance magnétique pendant ledit processus d'imagerie par résonance magnétique ; dans lequel le matériau d'extrusion est un matériau de co-extrusion qui comprend :
un premier composant de co-extrusion comprenant un polymère hydrophile qui comprend une substance ayant une pluralité de particules paramagnétiques, le premier composant de co-extrusion étant configuré de façon à renforcer la visibilité par résonance magnétique ; et
un second composant de co-extrusion comprenant un polymère structural, le second composant de co-extrusion étant configuré de façon à fournir un support structural.

7. Dispositif médical allongé selon la revendication 6, dans lequel la pluralité de particules paramagnétiques comprend de l'oxyde de fer super-magnétique.

8. Dispositif médical allongé selon la revendication 6, dans lequel la pluralité des particules paramagnétiques comprend de l'oxyde de dysprosium.
